# EUROPÄISCHE PATENTANMELDUNG

(11) **EP 4 810 505 A1**
(43) Veröffentlichungstag der Anmeldung: **23.09.2026**
(21) Anmeldenummer: 25164635.2
(22) Anmeldetag: 19.03.2025
(51) Int. Cl.: C07F 9/6574, C07C 45/50, C07C 47/02, C07C 47/21, C07F 15/00

(54) **PHOSPHITE UND DEREN EINSATZ IN HYDROFORMYLIERUNGSVERFAHREN**

(71) Anmelder: Evonik Oxeno GmbH & Co. KG, 45772 Marl (DE)
(72) Erfinder: FRANKE, Robert, 45772 Marl (DE); BÜKER, Julia, 44803 Bochum (DE); SALE, Anna Chiara, 40474 Düsseldorf (DE); BÖRNER, Armin, 18059 Rostock (DE); HOLZ, Jens, 18196 Kessin (DE); ROMEIKE, Kerstin, 18109 Rostock (DE); WENZEL, Gudrun, 18196 Kessin (DE)
(74) Vertreter: Evonik Patent Association

(57) **Zusammenfassung**

Phosphite und deren Einsatz in Hydroformylierungsverfahren.

## Beschreibung

Die Erfindung betrifft Phosphite und deren Einsatz in Hydroformylierungsverfahren.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die technische Aufgabe der Erfindung ist die Bereitstellung einer Verbindung, mit welcher bei der Hydroformylierung von Olefinen eine gute Ausbeute erzielt werden kann.

Die Aufgabe wird gelöst durch eine Verbindung gemäß Anspruch 1.

Verbindung gemäß einer der Formeln (1) bis (20):

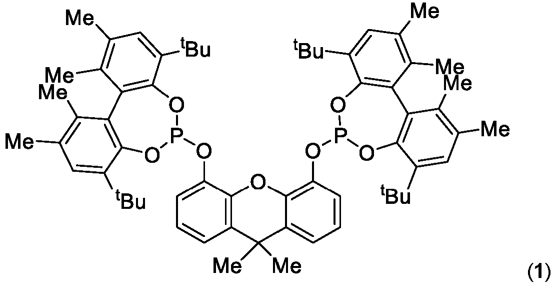

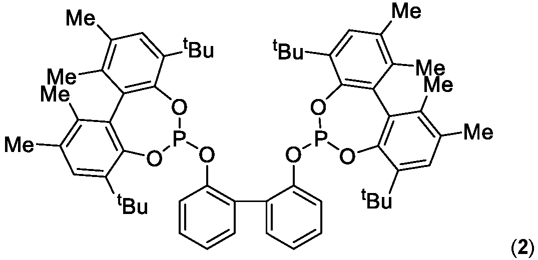

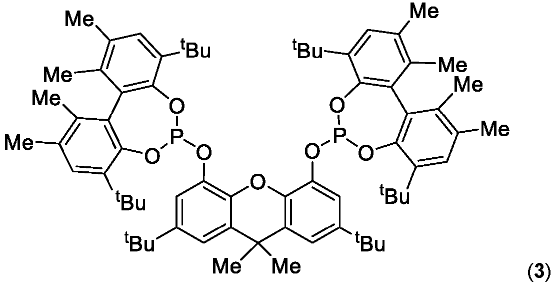

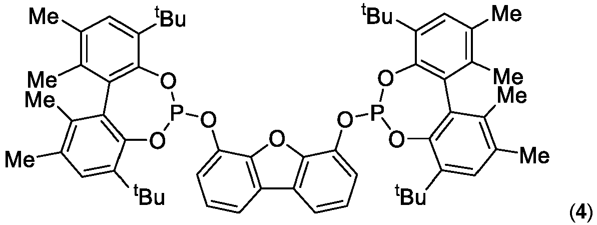

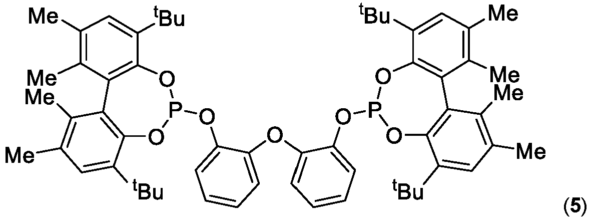

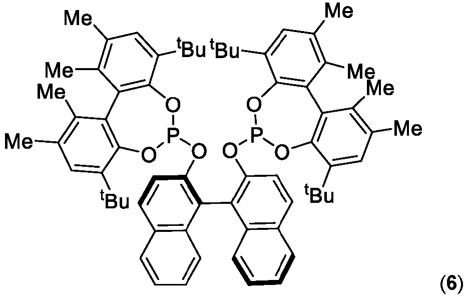

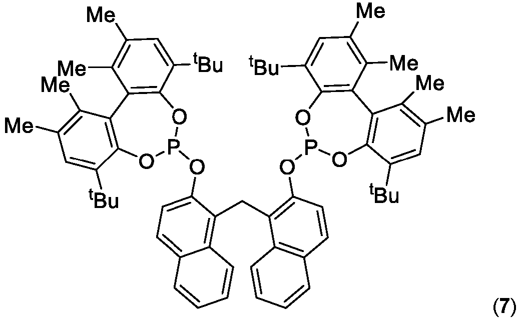

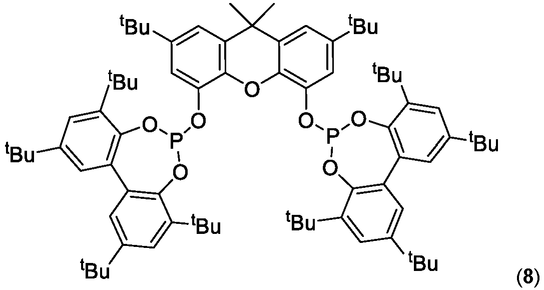

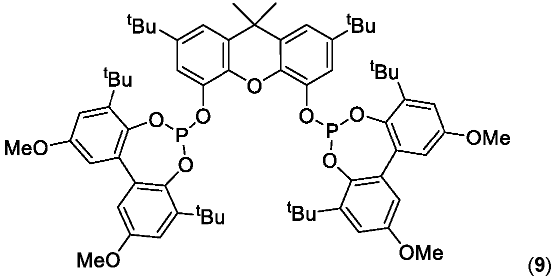

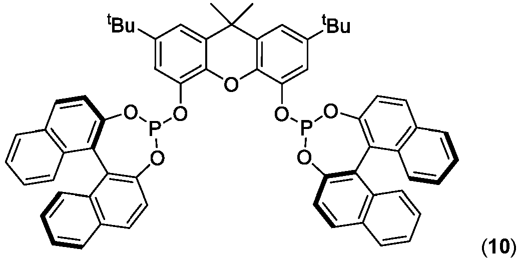

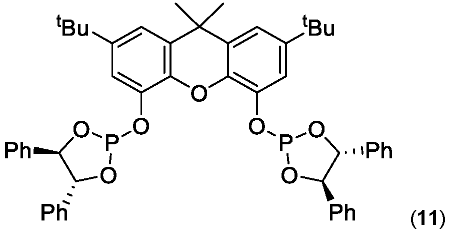

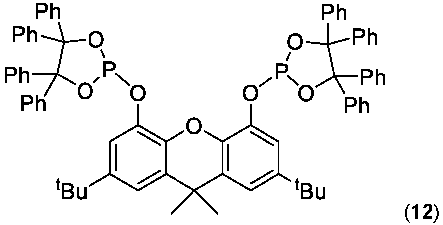

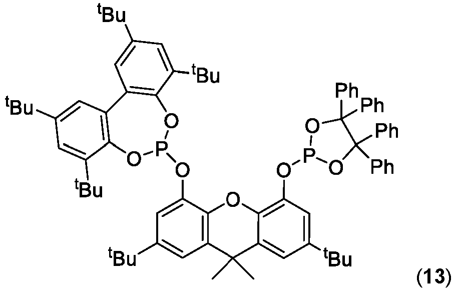

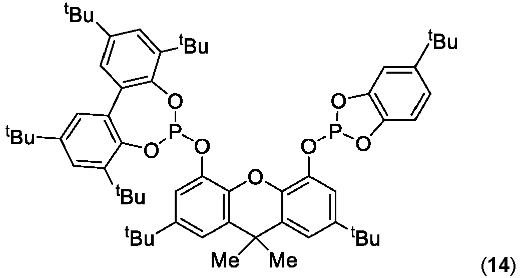

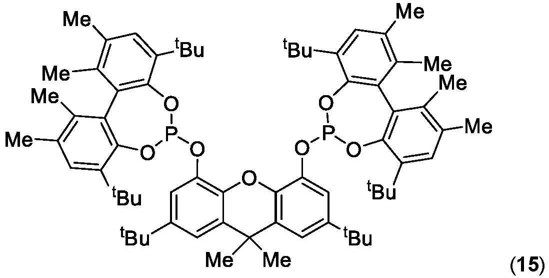

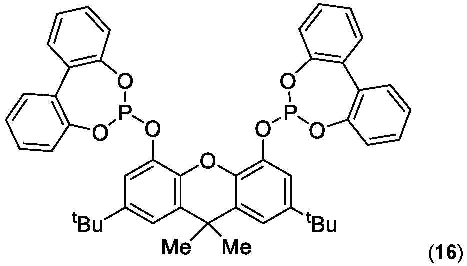

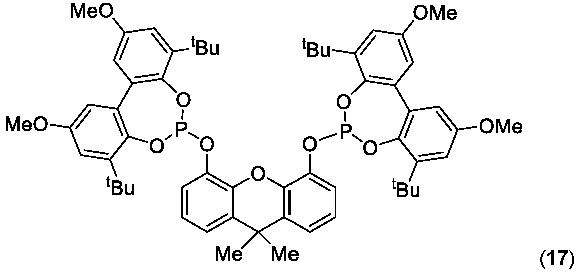

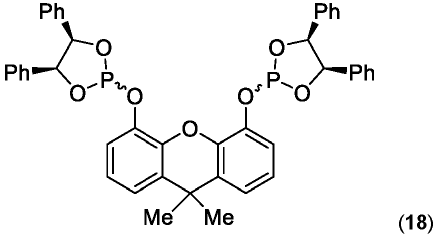

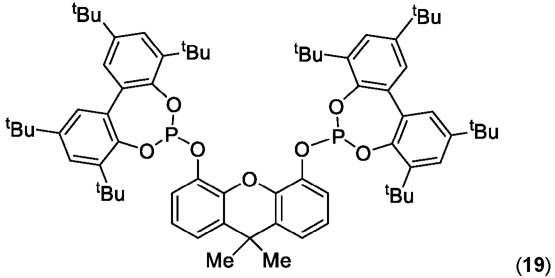

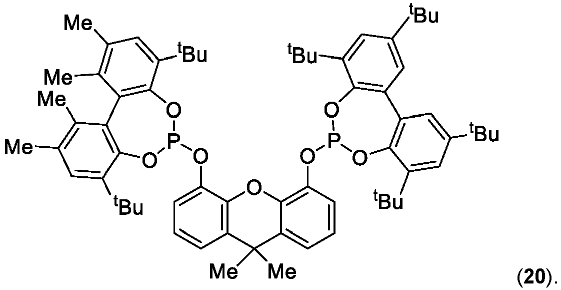

Im Folgenden soll die Erfindung anhand von Ausführungsbeispielen näher erläutert werden.

### Synthesen

### 6,6'-((9,9-Dimethyl-9H-xanthen-4,5-diyl)bis(oxy))bis(rac-4,8-di-tert-butyl-1,2,10,11-tetramethyldi-benzo[d,f][1,3,2]dioxaphosphepin) (Verbindung 1)

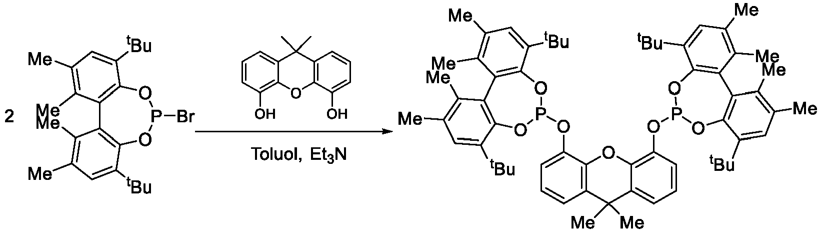

Zu einer Lösung des rac-6-Bromo-4,8-di-tert-butyl-1,2,10,11-tetramethyldibenzo[*d*,*f*[1,3,2]dioxaphosphepins (278 mg, 0.60 mmol) in Toluol (5 ml) gibt man

Triethylamin (91 mg, 0.90 mmol) und setzt anschließend 0.5 Äquivalente 9,9-Dimethyl-9H-xanthen-4,5-diol (73 mg, 0.30 mmol) hinzu. Nach dem Rühren bei Raumtemperatur (3 Tage) wird vom ausgefallenen Niederschlag abfiltriert und das Filtrat unter Vakuum eingeengt. Durch Säulenchromatographie (Eluent: Cyclohexan/AcOEt 99/1) lässt sich das Produkt als Diastereomerengemisch isolieren.

Ausbeute: 200 mg weißer Feststoff (66%), Smp. = 150-153°C.

³¹P-NMR (CD₂Cl₂): d +128.6 ppm (74%) und +128.2 ppm (26%), Gemisch aus *(R,R)*/*(S,S)-* und (R,S)-Verbindung.

¹H-NMR (CD₂Cl₂): *Diastereomer A,* d 7.22 (2H, br s, 2xHₐᵣ), 7.12 (2H, br s, 2xHₐᵣ), 7.08 (2H, dd, *J* 8.0, 1.4 Hz, 2xHₐᵣ), 6.77 (2H, dd, *J* 8.0, 8.0 Hz, 2xHₐᵣ), 6.24 (2H, m, 2xHₐᵣ), 2.28 (6H, s, 2xCH₃), 2.24 (6H, s, 2xCH₃), 1.83 (6H, s, 2xCH₃), 1.81 (6H, s, 2xCH₃), 1.51 (6H, s, 2xCH₃), 1.49 (18H, s, 2xC(CH₃)₃), 1.21 (18H, s, 2x C(CH₃)₃); *Diastereomer B,* d 7.33 (2H, br s, 2xHₐᵣ), 7.07 (2H, br s, 2xHₐᵣ), 6.97 (2H, dd, *J* 8.0, 1.5 Hz, 2xHₐᵣ), 6.37 (2H, dd, *J* 8.0, 8.0 Hz, 2xHₐᵣ), 5.60 (2H, m, 2xHₐᵣ), 2.34 (6H, s, 2xCH₃), 2.22 (6H, s, 2xCH₃), 1.77 (6H, s, 2xCH₃), 1.76 (6H, s, 2xCH₃), 1.51 (18H, s, 2xC(CH₃)₃), 1.49 (6H, s, 2xCH₃), 1.14 (18H, s, 2xC(CH₃)₃).

HRMS (ESI-TOF) [M+H]⁺: m/z berechnet: für C₆₃H₇₆O₇P₂ 1007.5145, gefunden: 1007.5150.

HRMS (ESI-TOF) [M+Na]⁺: m/z berechnet: für C₆₃H₇₆O₇P₂ 1029.4958, gefunden: 1029.4968.

### 2,2'-Bis((rac-4,8-di-tert-butyl-1,2,10,11-tetramethyldibenzo[d,f][1,3,2]dioxaphosphepin-6-yl)oxy)-1,1'-biphenyl (Verbindung 2)

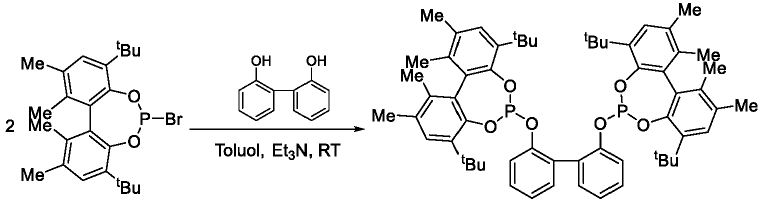

Zu einer Lösung des *rac*-6-Bromo-4,8-di-*tert*-butyl-1,2,10,11-tetramethyldibenzo[*d*,*f*][1,3,2]dioxaphos-phepins (834 mg, 1.80 mmol) in Toluol (5 ml) gibt man Triethylamin (273 mg, 2.7 mmol) und setzt anschließend 0.5 Äquivalente [1,1'-biphenyl]-2,2'-diol (168 mg, 0.90 mmol) hinzu. Nach dem Rühren bei Raumtemperatur über Nacht wird vom ausgefallenen Niederschlag abfiltriert und das Filtrat unter Vakuum eingeengt. Durch Säulenchromatographie (Eluent: Cyclohexan/AcOEt 99/1) wurde das Produkt als Diastereomerengemisch vorgereinigt und anschließend durch Umkristallisation aus Acetonitril in guter Reinheit erhalten.

Ausbeute: 690 mg weißer Feststoff (81%), Smp. = 167-170°C.

³¹P-NMR (CD₂Cl₂): d +131.2 ppm, Gemisch aus (*R,R*)/(*S,S*)*-* und (*R*,*S*)-Verbindung.

¹H-NMR (CD₂Cl₂): d *DiastereomerA (~60%),* d 7.22-7.10 (8H, m, 8xHₐᵣ), 7.04-6.83 (4H, m, 4xHₐᵣ), 2.32 (6H, s, 2xCH₃), 2.27 (6H, s, 2xCH₃), 1.86 (6H, s, 2xCH₃), 1.81 (6H, s, 2xCH₃), 1.35 (18H, s, 2xC(CH₃)₃), 1.19 (18H, s, 2xC(CH₃)₃); *Diastereomer B (~40%),* d 7.22-7.10 (8H, m, 8xHₐᵣ), 7.04-6.83 (4H, m, 4xHₐᵣ), 2.31 (6H, d 2xCH₃), 2.27 (6H, d 2xCH₃), 1.86 (6H, d, 2xCH₃), 1.81 (6H, d 2xCH₃), 1.33 (18H, s, 2xC(CH₃)₃), 1.19 (18H, s, 2xC(CH₃)₃).

HRMS (ESI-TOF) [M+H]⁺: m/z berechnet: für C₆₀H₇₂O₆P₂ 951.4882, gefunden: 951.4888.

HRMS (ESI-TOF) [M+Na]⁺: m/z berechnet: für C₆₀H₇₂O₆P₂ 973.4696, gefunden: 973.4709.

### 6,6'-((2,7-Di-tert-butyl-9,9-dimethyl-9H-xanthene-4,5-diyl)bis(oxy))bis(rac-4,8-di-tert-butyl-1,2,10,11-tetramethyldibenzo[d,t][1,3,2]dioxaphosphepin) (Verbindung 3)

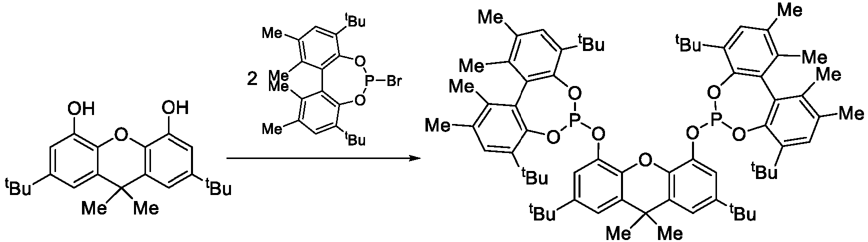

Zu einer Lösung des *rac*-6-Bromo-4,8-di-*tert*-butyl-1,2,10,11-tetramethyldibenzo[*d*,*f*][1,3,2]dioxa-phosphepins (835 mg, 1.80 mmol) in Toluol (7 ml) werden bei Raumtemperatur zunächst Triethylamin (273 mg, 2.70 mmol) und anschließend 2,7-Di-tert-butyl-9,9-dimethyl-9H-xanthene-4,5-diol (319 mg, 0.90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 95/5) gereinigt.

Ausbeute: 880 mg weißer Feststoff (87%), Smp. = 142-144°C.

³¹P-NMR (CD₂Cl₂): d +126.9 ppm (52%) und +126.6 ppm (48%), Gemisch aus *(R,R)*/*(S,S)-* und (R,S)-Verbindung.

¹H-NMR (CD₂Cl₂): *Diastereomer A,* d 7.35 (2H, s, 2xHₐᵣ), 7.09 (2H, s, 2xHₐᵣ), 6.96 (2H, d, *J* 2.2 Hz, 2xHₐᵣ), 5.90 (2H, d, *J* 2.1 Hz, 2xHₐᵣ), 2.30 (6H, s, 2xCH₃), 2.23 (6H, s, 2xCH₃), 1.80 (6H, s, 2xCH₃), 1.75 (6H, s, 2xCH₃), 1.53 (18H, s, 2xC(CH₃)₃), 1.17 (18H, s, 2xC(CH₃)₃), 1.09 (18H, s, 2xC(CH₃)₃), *Diastereomer B,* d 7.26 (2H, s, 2xHₐᵣ), 7.12 (2H, s, 2xHₐᵣ), 7.08 (2H, d, *J* 2.2 Hz, 2xHₐᵣ), 6.45 (2H, d, *J* 2.1 Hz, 2xHₐᵣ), 2.29 (6H, s, 2xCH₃), 2.25 (6H, s, 2xCH₃), 1.83 (6H, s, 2xCH₃), 1.82 (6H, s, 2xCH₃), 1.51 (6H, s, 2xCH₃), 1.22 (18H, s, 2xC(CH₃)₃), 1.14 (18H, s, 2xC(CH₃)₃).

¹³C-NMR (CD₂Cl₂): Diastereomerengemisch, d 128.8 (2xCₐᵣH), 128.7 (2xCₐᵣH), 128.1 (4xCₐᵣH), 119.7 (2xCₐᵣH), 118.6 (2xCₐᵣH), 118.0 (2xCₐᵣH), 117.7 (2xCₐᵣH), 35.3 (2x^{t}C), 35.2 (^{t}C),34.9 (^{t}C), 34.8 (^{t}C), 34.7 (^{t}C), 34.6 (^{t}C), 34.6 (^{t}C), 33.8 (2xCH₃), 33.6 (2xCH₃), 31.9 (6xCH₃), 31.8 (6xCH₃), 31.5 (m, 6xCH₃), 31.3 (6xCH₃), 31.2 (6xCH₃), 31.1 (m, 6xCH₃), 20.7 (2xCH₃), 20.6 (2xCH₃), 20.5 (2xCH₃), 20.5 (2xCH₃), 16.9 (2xCH₃), 16.8 (2xCH₃), 16.2 (4xCH₃).

HRMS (ESI-TOF) [M+H]⁺: m/z berechnet: für C₇₁H₉₂O₇P₂ 1119.6396, gefunden: 1119.6414.

HRMS (ESI-TOF) [M+Na]⁺: m/z berechnet: für C₇₁H₉₂O₇P₂ 1141.6211, gefunden: 1141.6232.

### 4,6-Bis((rac-4,8-di-tert-butyl-1,2,10,11-tetramethyldibenzo[d,f][1,3,2]dioxaphosphepin-6-yl)oxy)di-benzo[b,d]furan (Verbindung 4)

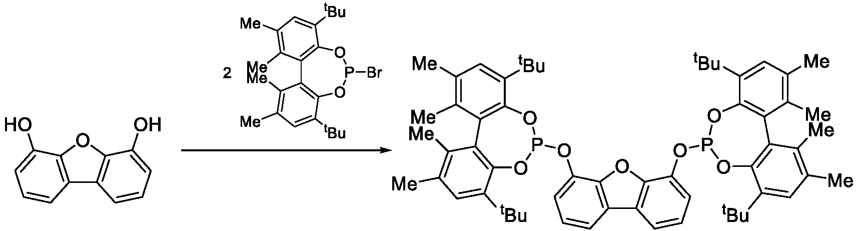

Zu einer Lösung des *rac*-6-Bromo-4,8-di-*tert*-butyl-1,2,10,11-tetramethyldibenzo[*d*,*f*][1,3,2]dioxa-phosphepins (835 mg, 1.80 mmol) in Toluol (7 ml) werden bei Raumtemperatur zunächst Triethylamin (273 mg, 2.70 mmol) und anschließend Dibenzo[*b*,*d*]furan-4,6-diol (180 mg, 0.90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 780 mg weißer Feststoff (90%), Smp. = 135-140°C.

³¹P-NMR (CD₂Cl₂): d +130.5 ppm (49%) und +130.3 ppm (51%), Gemisch aus (R,R)/(S,S)- und (R,S)-Verbindung.

¹H-NMR (CD₂Cl₂): ca. 1/1-Diastereomerengemisch, d 7.64 (2H, dd, *J* 7.8, 0.9 Hz, 2xHₐᵣ), 7.25+7.24 (2H, s, 2xHₐᵣ), 7.18+7.18 (2H, s, 2xHₐᵣ), 7.13 (2H, dt, *J* 7.9, 2.9 Hz, 2xHₐᵣ), 6.85 (2H, m, 2xHₐᵣ), 2.30 +2.29 + 2.28 (12H, 3xs, 4xCH₃), 1.85+1.84+1.84 (12H, 3xs, 4xCH₃), 1.46+1.46+1.34+1.32 (36H, 4xs, 4xC(CH₃)₃).

¹³C-NMR (CD₂Cl₂): ca. 1/1-Diastereomerengemisch; d 147.5+147.4 (4xCₐᵣ), 145.2+145.1 (4xCₐᵣ), 144.9 (4xCₐᵣ), 137.8-131.0 (restliche Cₐᵣ-Signale), 128.9+128.8 (4xCₐᵣH), 128.3 (4xCHₐᵣ), 126.9+126.9 (2xCₐᵣ), 123.7+123.6 (4xCHₐᵣ), 119.9+119.8 (4xCHₐᵣ), 116.4+116.3 (4xCHₐᵣ), 35.2 (2x^{t}C), 35.2 (2x^{t}C), 34.9 (2x^{t}C), 34.9 (2x^{t}C), 31.8 (12xCH₃), 31.3 (6xCH₃), 31.3 (6xCH₃), 20.5 (8xCH₃), 16.9 (2xCH₃), 16.8 (2xCH₃), 16.6 (4xCH₃).

HRMS (ESI-TOF) [M+H]⁺: m/z berechnet: für C₆₀H₇₀O₇P₂ 965.4675, gefunden: 965.4683.

HRMS (ESI-TOF) [M+Na]⁺: m/z berechnet: für C₆₀H₇₀O₇P₂ 987.4489, gefunden: 987.4501.

### 6,6'-((Oxybis(2,1-phenylen))bis(oxy))bis(4,8-di-tert-butyl-1,2,10,11-tetramethyldibenzo[d,f]-[1,3,2]dioxaphosphepin) (Verbindung 5)

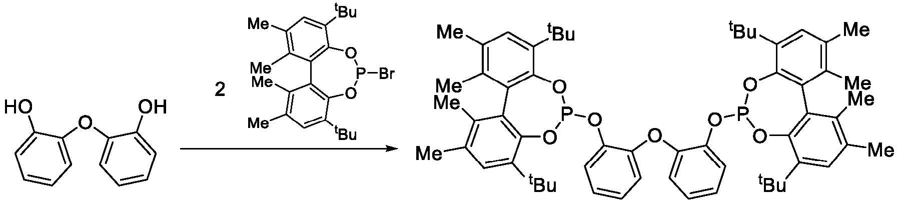

Zu einer Lösung des *rac*-6-Bromo-4,8-di-*tert*-butyl-1,2,10,11-tetramethyldibenzo[*d*,*f*][1,3,2]dioxa-phosphepins (835 mg, 1.80 mmol) in Toluol (7 ml) werden bei Raumtemperatur zunächst Triethylamin (273 mg, 2.70 mmol) und anschließend 2,2'-Oxodiphenol (182 mg, 0.90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 540 mg weißer Feststoff (62%), Smp. = 115-118°C.

³¹P-NMR (CD₂Cl₂): d +131.5 ppm (62%) und +131.3 ppm (38%), Gemisch aus (*R,R*)/(*S,S*)*-* und (*R*,*S*)-Verbindung.

¹H-NMR (CD₂Cl₂): Diastereomerengemisch, d 7.20-7.16 (4H, 3xs, 4xHₐᵣ), 7.03-6.89 (6H, m, 6xHₐᵣ), 6.83-6.77 (2H, m, 2xHₐᵣ), 2.29-2.24 (12H, 3xs, 4xCH₃), 1.86-1.81 (12H, 4xs, 4xCH₃), 1.35-1.32 (36H, 4xs, 4xC(CH₃)₃).

¹³C-NMR (CD₂Cl₂): Signale von 146-129 ppm nicht eindeutig zuordbar, 128.8+128.7 (4xCₐᵣH), 128.2 (4xCₐᵣH), 125.2+125.1 (4xCₐᵣH), 124.1 (4xCₐᵣH), 123.7 (4xCₐᵣH), 120.5+120.4 ppm (4xCₐᵣH), 35.2 (2x^{t}C), 35.1 (2x^{t}C), 34.9 (4x^{t}C), 31.8 (6xCH₃), 31.7 (6xCH₃), 31.4 (6xCH₃), 31.4 (6xCH₃), 20.5 (6xCH₃), 20.5 (2xCH₃), 16.9 (2xCH₃), 16.9 (2xCH₃), 16.6 (4xCH₃).

HRMS (ESI-TOF) [M+H]⁺: m/z berechnet: für C₆₀H₇₂O₇P₂ 967.4832, gefunden: 967.4845.

HRMS (ESI-TOF) [M+Na]⁺: m/z berechnet: für C₆₀H₇₂O₇P₂ 989.4645, gefunden: 989.4665.

### (R)-2,2'-Bis((rac-4,8-di-tert-butyl-1,2,10,11-tetramethyldibenzo[d,f][1,3,2]dioxaphosphepin-6-yl)oxy)-1,1'-binaphthalen (Verbindung 6)

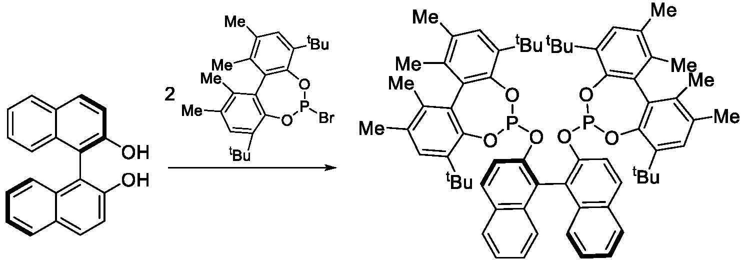

Eine Lösung von (*R*)-1,1'-Bi(2-naphthol) (229 mg, 0.80 mmol) und Triethylamin (243 mg, 2.40 mmol) in Toluol (3 ml) wird bei 0°C langsam mit einer Lösung des *rac*-6-Bromo-4,8-di-*tert*-butyl-1,2,10,11-tetramethyldibenzo[*d*,*f*][1,3,2]dioxa-phosphepins (788 mg, 1.70 mmol) in Toluol (5 ml) versetzt. Nach 30 Minuten lässt man auf Raumtemperatur erwärmen und die Reaktionsmischung zwei Tage weiterrühren. Anschließend wird vom ausgefallenen Niederschlag abfiltriert und das Filtrat unter Vakuum eingeengt. Die Reinigung erfolgte durch Säulenchromatographie (Eluent: Cyclohexan/AcOEt 99/1) und lieferte das Produkt als ein Gemisch aus drei Stereoisomeren.

Ausbeute: 550 mg weißer Feststoff (65%), Smp. = 120-122°C.

³¹P-NMR (CD₂Cl₂): d +133.7 ppm (38%), 23.4% +128.4 ppm (24%), 37.2% +125.0 ppm (38%) ppm; Gemisch aus (*S,R,S*)*-,* (*R,R,R*)*-* und (*S*,*R*,*R*)-Isomer.

¹H-NMR (CD₂Cl₂): *Diastereomerengemisch:* d 7.96 (1H, d, *J* 8.9 Hz, Hₐᵣ), 7.90-7.86 (2H, m, 2xHₐᵣ), 7.84 (1H, d, *J* 9.0 Hz, Hₐᵣ), 7.76-7.71 (2H, m, 2xHₐᵣ), 7.63 (1H, d, *J* 9.0 Hz, Hₐᵣ), 7.40-7.31 (2H, m, 2xHₐᵣ), 7.29-7.08 (4H, m, 4xHₐᵣ), 7.02-6.93 (3H, m, 3xHₐᵣ), 6.84 (1H, dd, *J* 8.9, 0.7 Hz, Hₐᵣ), 6.80 (1H, s, Hₐᵣ), 2.31 (3H, s, CH₃), 2.29 (CH₃), 2.22 (3H, s, CH₃), 2.21 (3H, s, CH₃), 2.21 (3H, s, CH₃), 2.20 (3H, s, CH₃), 1.86 (3H, s, CH₃), 1.85 (CH₃), 1.76 (3H, s, CH₃), 1.74 (3H, s, CH₃), 1.69 (3H, s, CH₃), 1.61 (3H, s, CH₃), 1.24 (9H, s, C(CH₃)₃), 1.21 (18H, s, 2xC(CH₃)₃), 1.10 (9H, s, C(CH₃)₃), 1.08 (9H, s, C(CH₃)₃), 1.05 (9H, s, C(CH₃)₃).

HRMS (ESI-TOF) [M+H]⁺: m/z berechnet: für C₆₈H₇₆O₆P₂ 1051.5190, gefunden: 1051.5214.

HRMS (ESI-TOF) [M+Na]⁺: m/z berechnet: für C₆₈H₇₆O₆P₂ 1073.5010, gefunden: 1073.5042.

### Bis(2-((rac-4,8-di-tert-butyl-1,2,10,11-tetramethyldibenzo[d,f][1,3,2]dioxaphosphepin-6-yl)oxy)naphthalen-1-yl)methane (Verbindung 7)

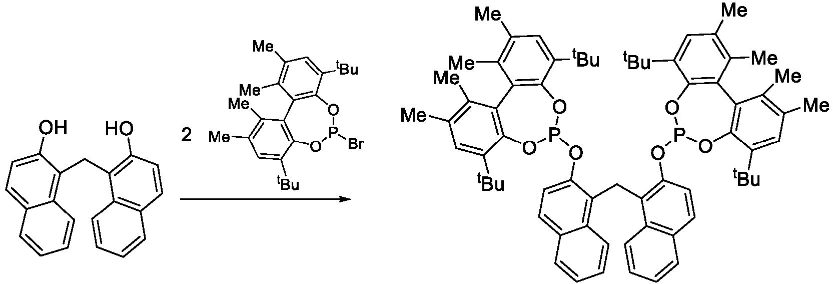

Eine Lösung von 1,1'-Methylenebis(naphthalen-2-ol) (240 mg, 0.80 mmol) und Triethylamin (243 mg, 2.40 mmol) in Toluol (3 ml) wird bei 0 °C langsam mit einer Lösung des rac-6-Bromo-4,8-di-tert-butyl-1,2,10,11-tetramethyldibenzo[*d*,*f*][1,3,2]dioxa-phosphepins (788 mg, 1.70 mmol) in Toluol (5 ml) versetzt. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Die Reinigung erfolgte durch Säulenchromatographie (Eluent: Cyclohexan/AcOEt 99/1) und lieferte das Produkt als ein Gemisch aus (*R,R*)*-*/(*S,S*)*-* und (R,S)-Stereoisomeren.

Ausbeute: 570 mg weißer Feststoff (65%), Smp. = 105-107°C.

³¹P-NMR (CD₂Cl₂): d +135.3 ppm (35%, *Diastereomer A*), +132.9 ppm (65%, *Diastereomer B*).

¹H-NMR (CD₂Cl₂): *Diastereomer A,* d 7.58 (2H, m, 2xHₐᵣ), 7.36 (2H, dd, J 8.9, 2.0 Hz, 2xHₐᵣ), 7.22 (2H, s, 2xHₐᵣ), 7.22-7.16 (4H, m, 4xHₐᵣ), 7.13 (2H, s, 2xHₐᵣ), 7.05 (2H, m, 2xHₐᵣ), 6.84 (2H, m, 2xHₐᵣ), 4.51 (2H, s, CH₂), 2.28 (6H, s, 2xCH₃), 1.88 (6H, s, 2xCH₃), 1.79 (6H, 2xCH₃), 1.50 (6H, 2xCH₃), 1.48 (18H, s, 2xC(CH₃)₃), 1.42 (18H, s, 2xC(CH₃)₃).; *Diastereomer B,* d 7.78 (2H, m, 2xHₐᵣ), 7.63 (2H, m, 2xHₐᵣ), 7.58 (4H, m, 4xHar), 7.25 (2H, dd, J 8.9 Hz, 1.1 Hz, 2xHₐᵣ), 7.22-7.16 (4H, m, 4xHₐᵣ), 7.08 (2H, m, 2xHₐᵣ), 4.96 (1H, AB-Sp., *J_{A,B}* 16.0 Hz, Hₐ-CH₂), 4.83 (1H, AB-Sp., *J_{A,B}* 16.0 Hz, H_{b}-CH₂), 2.28 (6H, s, 2xCH₃), 2.16 (6H, s, 2xCH₃), 1.84 (6H, 2xCH₃), 1.79 (6H, 2xCH₃), 1.51 (18H, s, 2xC(CH₃)₃), 1.32 (18H, s, 2xC(CH₃)₃).

HRMS (ESI-TOF) [M+H]⁺: m/z berechnet: für C₆₉H₇₈O₆P₂ 1065.5347, gefunden: 1065.5378.

HRMS (ESI-TOF) [M+Na]⁺: m/z berechnet: für C₆₉H₇₆O₆P₂ 1087.5166, gefunden: 1087.5198.

### 6,6'-((2,7-Di-tert-butyl-9,9-dimethyl-9H-xanthen-4,5-diyl)bis(oxy))bis(2,4,8,10-tetra-tert-butyldibenzo[d,f][1,3,2]dioxaphosphepin) (Verbindung 8)

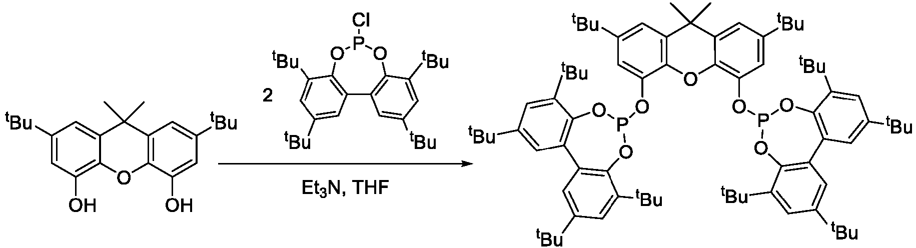

Zu einer Lösung von 2,7-Di-tert-butyl-9,9-dimethyl-9H-xanthen-4,5-diol (146 mg, 0.41 mmol) und Triethylamin (125 mg, 1.23 mmol) in THF (2 ml) wird unter Rühren und bei 0 °C das 2,4,8,10-Tetra-tert-butyl-6-chlorodibenzo[*d*,*f*][1,3,2]dioxaphosphepin (391 mg, 0.83 mmol) in THF (5 ml) langsam zugetropft und man lässt anschließend bei Raumtemperatur über Nacht weiterrühren. Zur Aufarbeitung wird vom ausgefallenem Hydrochlorid abfiltriert, das Lösemittel unter Vakuum entfernt und der Rückstand bei 60°C unter Vakuum getrocknet. Zur Reinigung wird das Rohprodukt in heißem Acetonitril gelöst. nach Abkühlung wird das ausgefallene Produkt abfiltriert und erneut unter Vakuum getrocknet.

Ausbeute: 295 mg weißer Feststoff (58%).

³¹P-NMR (CD₂Cl₂): d +132.9 ppm.

¹H-NMR (CD₂Cl₂): d 7.50 (4H, d, *J* 2.5 Hz, 4xHₐᵣ), 7.22 (4H, d, *J* 2.5 Hz, 4xHₐᵣ), 7.08 (2H, d, *J* 2.2 Hz, 2xHₐᵣ), 6.57 (2H, d, *J* 2.2 Hz, 2xHₐᵣ), 1.58 (6H, s, 2xCH₃), 1.43 (36H, s, 4xC(CH₃)₃), 1.38 (36H, s, 4xC(CH₃)₃), 1.14 (18H, s, 2xC(CH₃)₃).

¹³C-NMR (CD₂Cl₂): d 147.1 (4xCₐᵣ), 146.3 (m, 2xCₐᵣ), 145.7 (2xCₐᵣ), 140.9 (4xCₐᵣ), 139.5 (m, 4xCₐᵣ), 139.3 (2xCₐᵣ), 133.4 (4xCₐᵣ), 130.4 (2xCₐᵣ), 127.1 (4xCₐᵣH), 124.9 (4xCₐᵣH), 118.8 (2xCₐᵣH), 118.2 (2xCₐᵣH), 35.8 (4x^{t}C), 35.1 (^{t}C), 35.0 (4x^{t}C), 34.7 (2x^{t}C), 33.3 (2xCH₃), 31.7 (12xCH₃), 31.7 (6xCH₃), 31.3 (12xCH₃).

HRMS (ESI-TOF) [M+H]⁺: m/z berechnet: für C₇₉H₁₀₈O₇P₂ 1231.7649, gefunden: 1231.7677.

| | | | | |
|---|---|---|---|---|
| Elementaranalyse: | berechnet: | C 77.04% | H 8.84% | P 5.03% |
| | gefunden: | C 77.20% | H 8.96% | P 4.95% |

### 6,6'-((2,7-Di-tert-butyl-9,9-dimethyl-9H-xanthen-4,5-diyl)bis(oxy))bis(4,8-di-tert-butyl-2,10-dimethoxydibenzo[d,t][1,3,2]dioxaphosphepin) (Verbindung 9)

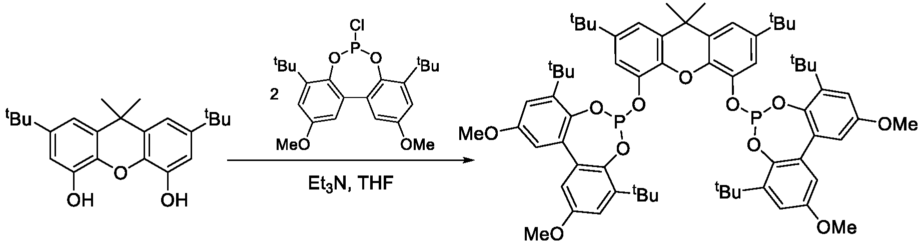

Zu einer Lösung von 2,7-Di-*tert*-butyl-9,9-dimethyl-9H-xanthen-4,5-diol (224 mg, 0.63 mmol) und Triethylamin (192 mg, 1.89 mmol) in THF (2 ml) wird unter Rühren und bei 0°C das 4,8-Di-tert-butyl-6-chlor-2,10-dimethoxydibenzo[*d*,*f*][1,3,2]dioxaphosphepin (534 mg, 1.26 mmol) in THF (7 ml) langsam zugetropft und man lässt anschließend bei Raumtemperatur über Nacht weiterrühren. Zur Aufarbeitung wird vom ausgefallenem Hydrochlorid abfiltriert, das Lösemittel unter Vakuum entfernt und der Rückstand bei 60°C unter Vakuum getrocknet. Zur Reinigung wird das Rohprodukt in heißem Acetonitril gelöst, nach Abkühlung wird das ausgefallene Produkt abfiltriert und erneut unter Vakuum getrocknet.

Ausbeute: 405 mg weißer Feststoff (57%).

³¹P-NMR (CD₂Cl₂): d +135.0 ppm.

¹H-NMR (CD₂Cl₂): d 7.07-6.99 (6H, m, 6xHₐᵣ), 6.70 (2H, d, *J* 3.1 Hz, 2xHₐᵣ), 6.25 (2H, d, *J* 2.1 Hz, 2xHₐᵣ), 3.80 (12H, s, 4xOCH₃), 1.53 (6H, s, 2xCH₃), 1.42 (36H, s, 4xC(CH₃)₃), 1.13 (18H, s, 2xC(CH₃)₃).

¹³C-NMR (CD₂Cl₂): d 156.3 (4xCₐᵣ-OMe), 145.6 (2xCₐᵣ), 143.2 (4xCₐᵣ), 142.2 (m, 2xCₐᵣ), 139.8 (m, 4xCₐᵣ), 138.8 (2xCₐᵣ), 134.3 (4xCₐᵣ), 129.8 (2xCₐᵣ), 118.4 (2xCₐᵣH), 118.2 (2xCₐᵣH), 114.7 (4xCₐᵣH), 113.4 (4xCₐᵣH), 55.9 (4xOCH₃), 35.8 (4x^{t}C), 34.9 (^{t}C), 34.7 (2x^{t}C), 33.7 (2xCH₃), 31.5 (6xCH₃), 31.1 (12xCH₃).

HRMS (ESI-TOF) [M+H]⁺: m/z berechnet: für C₆₇H₈₄O₁₁P₂ 1127.5566, gefunden: 1127.5580.

| | | | | |
|---|---|---|---|---|
| Elementaranalyse: | berechnet: | C 71.38% | H 7.51% | P 5.50% |
| | gefunden: | C 71.33% | H 7.41% | P 5.39% |

### (R,R)-4,4'-((2,7-di-tert-butyl-9,9-dimethyl-9H-xanthen-4,5-diyl)bis(oxy))didinaphtho[2,1-d:1',2'-f][1,3,2]dioxaphosphepin (Verbindung 10)

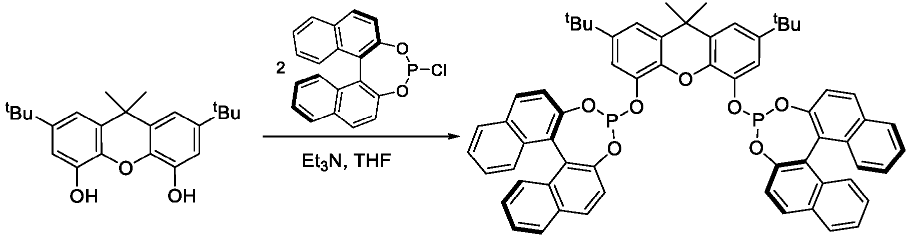

Zu einer Lösung von 2,7-Di-*tert*-butyl-9,9-dimethyl-9H-xanthene-4,5-diol (242 mg, 0.68 mmol) und Triethylamin (207 mg, 2.04 mmol) in THF (3 ml) wird unter Rühren und bei 0°C das (R)-4-chlorodinaphtho[2,1-*d*:1',2'-*f*][1,3,2]dioxaphosphepin (534 mg, 1.26 mmol) in THF (7 ml) langsam zugetropft und man lässt anschließend bei Raumtemperatur über Nacht weiterrühren. Nach Abtrennung des ausgefallenen Hydrochlorids wird das Lösemittel unter Vakuum entfernt und der Rückstand bei 60°C unter Vakuum getrocknet. Zur Reinigung wird das Rohprodukt mittels Säulenchromatographie (Eluent: Heptan/Dichlormethan 1/1) gereinigt.

Ausbeute: 190 mg weißer Feststoff (28%).

³¹P-NMR (CD₂Cl₂): d +141.7 ppm (Produkt).

¹H-NMR (CD₂Cl₂): d 7.92-7.84 (4H, m, 4xHₐᵣ), 7.82 (2H, br d, *J* 8.8 Hz, 2xHₐᵣ), 7.71 (2H, br d, *J* 8.9 Hz, 2xHₐᵣ), 7.50 (2H, br d, *J* 8.8 Hz, 2xHₐᵣ), 7.47-7.38 (6H, m, 6xHₐᵣ), 7.34-7.21 (10H, m, 10xHₐᵣ), 7.50 (2H, br d, *J* 2.4 Hz, 2xHₐᵣ), 1.72 (6H, s, 2xCH₃), 1.21 (18H, s, 2xC(CH₃)₃).

¹³C-NMR (CD₂Cl₂): d 148.5 (2xCₐᵣ), 147.7 (4xCₐᵣ), 146.6 (2xCₐᵣ), 139.9 (2xCₐᵣ), 139.1 (4xCₐᵣ), 133.2 (2xCₐᵣ), 132.9 (2xCₐᵣ), 132.0 (2xCₐᵣ), 131.6 (2xCₐᵣ), 131.2 (2xCₐᵣ), 130.8 (2xCₐᵣH), 130.3 (2xCₐᵣH), 128.8 (4xCₐᵣH), 127.2 (4xCₐᵣH), 126.6 (2xCₐᵣH), 126.6 (2xCₐᵣH), 125.4 (2xCₐᵣH), 125.4 (2xCₐᵣH), 123.1 (4xCₐᵣ), 122.5 (2xCₐᵣH), 122.4 (2xCₐᵣH), 119.2 (2xCₐᵣH), 118.8 (2xCₐᵣH), 35.4 (^{t}C), 34.8 (2x^{t}C), 33.0 (2xCH₃), 31.4 (6xCH₃).

HRMS (ESI-TOF) [M+H]⁺: m/z berechnet: für C₆₃H₅₂O₇P₂ 983.3267, gefunden: 983.3287.

| | | | | |
|---|---|---|---|---|
| Elementaranalyse: | berechnet: | C 76.97% | H 5.33% | P 6.30% |
| | gefunden: | C 76.80% | H 5.61% | P 6.12% |

### (4R,4'R,5R,5'R)-2,2'-((2,7-Di-tert-butyl-9,9-dimethyl-9H-xanthen-4,5-diyl)bis(oxy))bis(4,5-diphenyl-1,3,2-dioxaphospholan) (Verbindung 11)

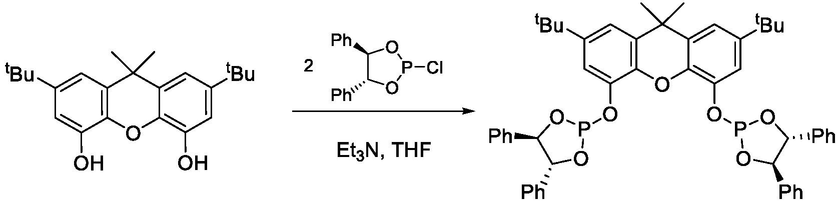

Zu einer Lösung von 2,7-Di-*tert*-butyl-9,9-dimethyl-9*H*-xanthen-4,5-diol (323 mg, 0.91 mmol) und Triethylamin (277 mg, 2.74 mmol) in THF (3 ml) wird unter Rühren und bei 0°C das (4R,5R)-2-chloro-4,5-diphenyl-1,3,2-dioxaphospholan (508 mg, 1.82 mmol) in THF (7 ml) langsam zugetropft und man lässt anschließend bei Raumtemperatur über Nacht weiterrühren. Nach Abtrennung des Hydrochlorids wird das Lösemittel unter Vakuum entfernt und der Rückstand bei 60°C unter Vakuum getrocknet. Zur Reinigung wird das Rohprodukt mittels Säulenchromatographie (Eluent: Heptan/AcOEt 3/1) gereinigt. Die isolierte Fraktion besteht aus einem Gemisch des Produkts und seinem Monophosphit. Die Abtrennung des letzteren gelingt durch Zugabe von Hexan, in dem das Monophosphit in Lösung geht. Das verbleibende Bisphosphit (weißer Feststoff) wird abfiltriert und getrocknet.

Ausbeute: 245 mg weißer Feststoff (32%).

³¹P-NMR (CD₂Cl₂): d +134.1 ppm.

¹H-NMR (CD₂Cl₂): d 7.34-7.11 (24H, m, 24xHₐᵣ), 5.29 (2H, br d, *J* 9.5 Hz, 2xCH-O), 4.82 (2H, br d, *J* 9.5 Hz, 2xCH-O), 1.71 (6H, s, 2xCH₃), 1.34 (18H, s, 2xC(CH₃)₃).

¹³C-NMR (CD₂Cl₂): d 146.6 (2xCₐᵣ), 140.3 (2xCₐᵣ), 139.7 (2xCₐᵣ), 136.9 (2xCₐᵣ), 136.8 (m, 2xCₐᵣ), 131.6 (m, 2xCₐᵣ), 128.9 (2xCₐᵣH), 128.9 (4xCₐᵣH), 128.8 (4xCₐᵣH), 128.8 (2xCₐᵣH), 127.9 (4xCₐᵣH), 127.4 (4xCₐᵣH), 118.3 (2xCₐᵣH), 118.0 (m, 2xCₐᵣH), 87.5 (m, 2xCH-O), 84.1 (2x, m, CH-O), 35.5 (^{t}C), 34.4 (2x^{t}C), 32.6 (2xCH₃), 31.6 (6xCH₃).

HRMS (ESI-TOF) [M+H]⁺: m/z berechnet: für C₅₁H₅₂O₇P₂ 839.3267, gefunden: 839.3277.

| | | | | |
|---|---|---|---|---|
| Elementaranalyse: | berechnet: | C 73.02% | H 6.25% | P 7.38% |
| | gefunden: | C 73.22% | H 6.09% | P 7.15% |

### 2,2'-((2,7-Di-tert-butyl-9,9-dimethyl-9H-xanthen-4,5-diyl)bis(oxy))bis(4,4,5,5-tetraphenyl-1,3,2-dioxaphospholan) (Verbindung 12)

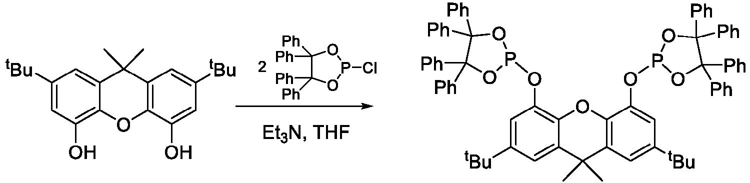

Zu einer Lösung von 248 mg 2,7-Di-tert-butyl-9,9-dimethyl-9H-xanthen-4,5-diol (0.70 mmol) und Triethylamin (214 mg, 2.11 mmol) in THF (6 ml) tropft man unter Rühren bei 0°C (Eisbad) eine Lösung von 2-Chloro-4,4,5,5-tetraphenyl-1,3,2-dioxaphospholan (608 mg, 1.41 mmol) in THF (3 ml) hinzu. Man lässt über Nacht bei Raumtemperatur rühren und entfernt anschließend den ausgefallenen Niederschlag durch Filtration unter anaeroben Bedingungen. Im Filtrat wurde nur ein geringer Teil des Produkts erhalten, da dieses offenbar schlecht löslich in THF ist. Zur weiteren Aufarbeitung wurde der verbliebene Feststoff auf der Fritte mit Toluol (10 ml) verrührt, um das Triethylammoniumchlorid abzutrennen. Nach Einengen des Filtrats unter Vakuum wird der erhaltene Rückstand unter Vakuum bei 60°C getrocknet.

Ausbeute: 115 mg weißer Feststoff (14%).

³¹P-NMR (CD₂Cl₂): d +136.9 ppm.

¹H-NMR (CD₂Cl₂): d 7.71-7.64 (8H, m, 8xHₐᵣ), 7.22-7.01 (26H, m, 26xHₐᵣ), 6.96-6.87 (8H, m, 6xHₐᵣ), 5.97 (2H, br d, *J* 2.4 Hz, 2xHₐᵣ), 1.58 (6H, s, 2xCH₃), 1.19 (18H, s, 6xCH₃).

¹³C-NMR (CD₂Cl₂): d 146.2 (2xCₐᵣ-O), 143.3 (4xCₐᵣ), 142.4 (4xCₐᵣ), 140.0 (2xCₐᵣ-O), 137.1 (2xCₐᵣ), 130.8 (2xCₐᵣ), 130.6 (8xCₐᵣH), 129.2 (8xCₐᵣH), 127.7 (4xCₐᵣH), 127.7 (8xCₐᵣH), 127.3 (8xCₐᵣH), 127.3 (4xCₐᵣH), 119.3 (2xCₐᵣH), 118.7 (2xCₐᵣH) 95.5 (m, 4x^{t}C-O), 35.2 (^{t}C), 34.8 (2x^{t}C), 32.8 (2xCH₃), 31.5 (6xCH₃).

HRMS (ESI-TOF) [M+Na]⁺: m/z berechnet: für C₇₅H₆₈O₇P₂ 1165.4337, gefunden: 1165.4357.

### 2,7-Di-tert-butyl-9,9-dimethyl-5-((2,4,8,10-tetra-tert-butyldibenzo[d,f][1,3,2]dioxaphosphepin-6-yl)oxy)-9H-xanthen-4-ol

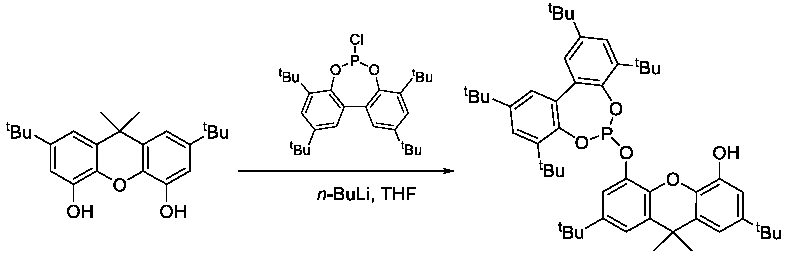

Eine Lösung von 393 mg 2,7-Di-*tert*-butyl-9,9-dimethyl-9*H*-xanthen-4,5-diol (1.11 mmol) in THF (4 ml) wird unter Rühren bei -20 °C mit einer n-Butyllithium-Lösung (0.53 M in Hexan, 2.1 ml) langsam versetzt. Nach 20 Minuten wird das Kältebad entfernt und eine Lösung des 2,4,8,10-Tetra-*tert*-butyl-6-chlorodibenzo[*d*,*f*][1,3,2]dioxaphosphepins (527 mg, 1.1 mmol) in THF (3 ml) tropfenweise hinzugefügt. Man lässt über Nacht bei Raumtemperatur rühren und entfernt anschließend die Lösemittel unter Vakuum. Der feste Rückstand wird in Toluol (8 ml) aufgenommen und über eine G4-Fritte filtriert. Nach dem Entfernen des Toluols im Filtrat wird der Rest mittels Säulenchromatographie (Eluent Heptan/CH₂Cl₂ 4/1) gereinigt.

Ausbeute: 425 mg weißer Feststoff (48%).

³¹P-NMR (CD₂Cl₂): d +137.3 ppm.

¹H-NMR (CD₂Cl₂): d 7.53 (2H, d, *J* 2.5 Hz, 2xHₐᵣ), 7.27 (2H, d, *J* 2.5 Hz, 2xHₐᵣ), 7.22 (1H, m, Hₐᵣ), 6.98-6.95 (2H, m, 2xHₐᵣ), 6.90 (1H, d, *J* 2.2 Hz, Hₐᵣ), 5.58 (1H, d, *J* 1.8 Hz, OH), 1.65 (6H, s, 2xCH₃), 1.46 (18H, s, 2xC(CH₃)₃), 1.41 (18H, s, 2xC(CH₃)₃), 1.33 (18H, s, C(CH₃)₃), 1.27 (18H, s, C(CH₃)₃).

¹³C-NMR (CD₂Cl₂): d 147.6 (2xCₐᵣ), 147.0 (Cₐᵣ), 146.6 (Cₐᵣ), 145.6 (d, *J* 6.0 Hz, Cₐᵣ), 143.9 (Cₐᵣ), 140.8 (d, *J* 1.7 Hz, 2xCₐᵣ), 139.4 (d, *J* 2.4 Hz, 2xCₐᵣ), 139.0 (Cₐᵣ), 135.9 (Cₐᵣ), 133.2 (d, *J* 3.9 Hz, 2xCₐᵣ), 131.7 (Cₐᵣ), 130.0 (Cₐᵣ), 127.1 (2xCₐᵣH), 125.1 (2xCₐᵣH), 118.7 (CₐᵣH), 117.9 (d, *J* 5.7 Hz, CₐᵣH), 113.8 (CₐᵣH), 111.0 (CₐᵣH), 35.8 (2x^{t}C), 35.4 (^{t}C), 35.1 (2x^{t}C), 35.0 (^{t}C), 34.9 (^{t}C), 32.4 (2xCH₃), 31.7 (6xCH₃), 31.7 (3xCH₃), 31.6 (3xCH₃), 31.3 (d, *J* 2.5 Hz, 6xCH₃).

HRMS (ESI-TOF) [M+Na]⁺: m/z berechnet: für C₅₁H₆₉O₅P 815.4780, gefunden: 815.4791.

HRMS (ESI-TOF) [M+Na+O]⁺: m/z berechnet: für C₅₁H₆₉O₅P 831.4728, gefunden: 831.4728.

### 2,4,8,10-Tetra-tert-butyl-6-((2,7-di-tert-butyl-9,9-dimethyl-5-((4,4,5,5-tetraphenyl-1,3,2-dioxaphospholan-2-yl)oxy)-9H-xanthen-4-yl)oxy)dibenzo[d,t][1,3,2]dioxaphosphepin (Verbindung 13)

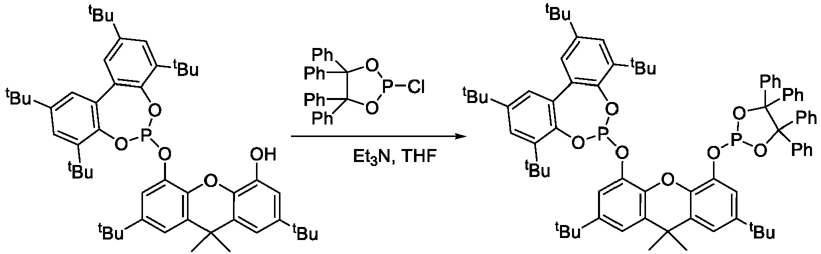

Zu einer Lösung von 388 mg 2,7-Di-*tert*-butyl-9,9-dimethyl-5-((2,4,8,10-tetra-*tert*-butyldi-benzo[*d*,*f*][1,3,2]dioxaphosphepin-6-yl)oxy)-9*H*-xanthen-4-ol (0.49 mmol) und Triethylamin (74 mg, 0.73 mmol) in THF (3 ml) tropft man unter Rühren bei 0°C (Eisbad) eine Lösung von 2-Chloro-4,4,5,5-tetraphenyl-1,3,2-dioxaphospholan (211 mg, 0.49 mmol) in THF (3 ml) hinzu. Man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand in Acetonitril (7 ml) aufgenommen und bei Siedehitze gelöst. Der beim Abkühlen rasch ausgefallene Niederschlag wird abgetrennt und unter Vakuum bei 60°C getrocknet.

Ausbeute: 395 mg weißer Feststoff (68%).

³¹P-NMR (CD₂Cl₂): d +137.58 und +131.3 ppm (2xd, *J* 35 Hz).

¹H-NMR (CD₂Cl₂): d 7.64-7.58 (4H, m, 4xHₐᵣ), 7.51 (2H, d, *J* 2.5 Hz, 2xHₐᵣ), 7.24 (2H, d, *J* 2.5 Hz, 2xHₐᵣ), 7.20-6.95 (18H, m, 18xHₐᵣ), 6.65 (1H, m, Hₐᵣ), 6.05 (1H, m, Hₐᵣ), 1.59 (6H, s, 2xCH₃), 1.50 (18H, s, 2xC(CH₃)₃), 1.33 (18H, s, 2xC(CH₃)₃), 1.20 (9H, s, C(CH₃)₃), 1.14 (9H, s, C(CH₃)₃)

¹³C-NMR (CD₂Cl₂): d 147.1-130.8 (5xCₐᵣ-O, 9xCₐᵣ), 130.5-127.1 (40xCₐᵣH), 124.9 (4xCₐᵣ), 119.0 (CₐᵣH), 119.0 (d, *J* 5.4 Hz, CₐᵣH), 118.2 (CₐᵣH), 117.9 (CₐᵣH), 95.0 (d, *J* 7.9 Hz, 2xC-O), 35.9 (2x^{t}C), 35.3 (^{t}C), 34.9 (2x^{t}C), 34.8 (^{t}C), 34.7 (^{t}C), 32.5 (2xCH₃), 31.7 (6xCH₃), 31.7 (3xCH₃), 31.6 (3xCH₃), 31.5 (3xCH₃), 31.4 (d, *J* 2.5 Hz, 6xCH₃).

HRMS (ESI-TOF) [M+Na]⁺: m/z berechnet: für C₇₇H₈₈O₇P₂ 1209.5903, gefunden: 1209.5917.

| | | | | |
|---|---|---|---|---|
| Elementaranalyse: | berechnet: | C 77.88% | H 7.47% | P 5.22% |
| | gefunden: | C 77.46% | H 7.61% | P 5.27% |

### 2,4,8,10-Tetra-tert-butyl-6-((2,7-di-tert-butyl-5-((5-(tert-butyl)benzo[d][1,3,2]dioxaphosphol-2-yl)oxy)-9,9-dimethyl-9H-xanthen-4-yl)oxy)dibenzo[d,f][1,3,2]dioxaphosphepin (Verbindung 14)

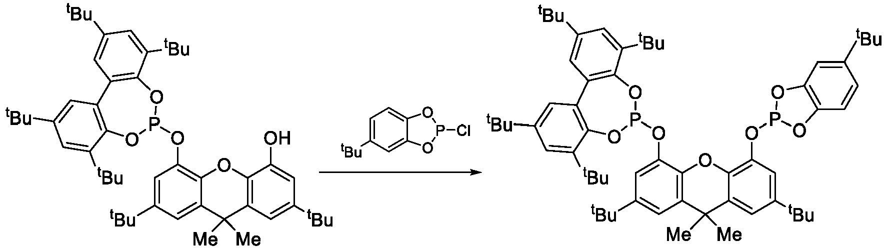

Zu einer Lösung des 2,7-Di-*tert*-butyl-9,9-dimethyl-5-((2,4,8,10-tetra-tert-butyldibenzo[*d*,*f*][1,3,2]di-oxaphosphepin-6-yl)oxy)-9*H*-xanthen-4-ols (198 mg, 0.25 mmol) in Toluol (3 ml) gibt man bei 0°C zunächst Triethylamin (38 mg, 0.37 mmol) und anschließend eine Lösung des 5-(*tert-*Butyl)-2-chlorobenzo[*d*][1,3,2]dioxaphosphols (63 mg, 0.28 mmol) in Toluol (2 ml) hinzu. Nach ca. 30 Minuten lässt man auf Raumtemperatur erwärmen und rührt über Nacht weiter. Nach der Filtration und Einengen des Filtrats wird der verbleibende Rückstand in n-Hexan (2 ml) gelöst und im Kühlschrank aufbewahrt. Nur eine sehr geringe Menge Niederschlag (30 mg) fiel aus, so dass die Mutterlauge eingeengt wurde und das Bisphosphit in einer Reinheit von etwa 95% erhalten wurde.

Ausbeute: 180 mg weißer Feststoff (73%), Smp. = 138-140°C.

³¹P-NMR (CD₂Cl₂): d +132.8 ppm (d, *J* 15.3 ppm) und +126.9 ppm (d, *J* 15.3 Hz).

¹H-NMR (CD₂Cl₂): d 7.51 (1H, d, *J* 2.4 Hz, Hₐᵣ), 7.49 (1H, d, *J* 2.5 Hz, Hₐᵣ), 7.24 (1H, d, *J* 2.4 Hz, Hₐᵣ), 7.23 (1H, d, *J* 2.4 Hz, Hₐᵣ), 7.09 (1H, dd, *J* 2.2, 0.5 Hz, Hₐᵣ), 7.05 (1H, dd, *J* 2.2, 0.5 Hz, Hₐᵣ), 6.93 (1H, m, Hₐᵣ), 6.84 (1H, m, Hₐᵣ), 6.84 (1H, m, Hₐᵣ), 6.82 (1H, m, Hₐᵣ), 6.73 (1H, dd, *J* 2.3, 1.5 Hz, Hₐᵣ), 1.54 (3H, s, CH₃), 1.52 (3H, s, CH₃), 1.47 (9H, s, C(CH₃)₃), 1.43 (9H, s, C(CH₃)₃), 1.38 (9H, s, C(CH₃)₃), 1.37 (9H, s, C(CH₃)₃), 1.21 (9H, s, C(CH₃)₃), 1.17 (9H, s, C(CH₃)₃), 1.15 (9H, s, C(CH₃)₃).

¹³C-NMR (CD₂Cl₂): d 147.2 (Cₐᵣ), 147.1 (Cₐᵣ), 146.6 (Cₐᵣ), 146.1 (Cₐᵣ), 146.0 (Cₐᵣ), 145.4 (d, *J* 7.4 Hz, Cₐᵣ), 143.1 (d, *J* 7.3 Hz, Cₐᵣ), 141.0 (Cₐᵣ), 140.9 (Cₐᵣ), 139.5 (Cₐᵣ), 139.4 (Cₐᵣ), 139.2 (d, *J* 6.7 Hz, Cₐᵣ), 138.1 (d, *J* 3.8 Hz, Cₐᵣ), 133.4 (d, *J* 4.1 Hz, Cₐᵣ), 133.3 (d, *J* 3.5 Hz, Cₐᵣ), 131.0 (Cₐᵣ), 130.7 (Cₐᵣ), 129.4 (Cₐᵣ), 128.6 (Cₐᵣ), 127.0 (2xCₐᵣH), 125.0 (CₐᵣH), 125.0 (CₐᵣH), 119.6 (CₐᵣH), 119.0 (d, *J* 4.1 Hz, CₐᵣH), 118.9 (CₐᵣH), 118.2 (CₐᵣH), 118.1 (d, *J* 4.1 Hz, CₐᵣH), 111.5 (CₐᵣH), 110.0 (CₐᵣH), 35.9 (^{t}C), 35.8 (^{t}C), 35.9 (^{t}C), 35.1 (^{t}C), 35.0 (2x^{t}C), 34.8 (^{t}C), 34.7 (^{t}C), 34.7 (^{t}C), 32.8 (CH₃), 32.6 (CH₃), 31.7 (9xCH₃), 31.6 (3xCH₃), 31.5 (3xCH₃), 31.4 (d, *J* 2.1 Hz, 3xCH₃), 31.3 (d, J 2.5 Hz, 3xCH₃).

HRMS (ESI-TOF) [M+Na]⁺: m/z berechnet: für C₆₁H₈₀O₇P₂ 1009.5271, gefunden: 1009.5303.

HRMS (ESI-TOF) [M+Na+O]⁺: m/z berechnet: für C₆₁H₈₀O₇P₂ 1025.5221, gefunden: 1025.5197.

### 6,6'-((2,7-Di-tert-butyl-9,9-dimethyl-9H-xanthene-4,5-diyl)bis(oxy))bis(rac-4,8-di-tert-butyl-1,2,10,11-tetramethyldibenzo[d,t][1,3,2]dioxaphosphepin) (Verbindung 15)

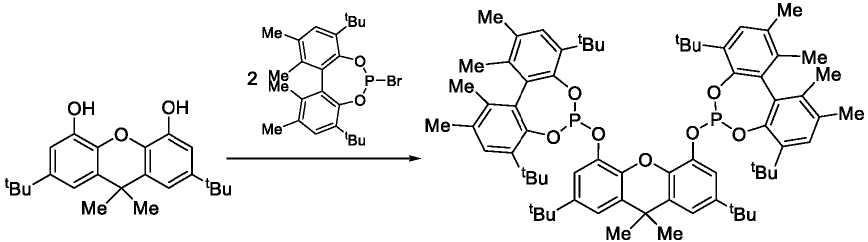

Zu einer Lösung des *rac*-6-Bromo-4,8-di-*tert*-butyl-1,2,10,11-tetramethyldibenzo[*d*,*f*][1,3,2]dioxa-phosphepins (835 mg, 1.80 mmol) in Toluol (7 ml) werden bei Raumtemperatur zunächst Triethylamin (273 mg, 2.70 mmol) und anschließend 2,7-Di-tert-butyl-9,9-dimethyl-9H-xanthene-4,5-diol (319 mg, 0.90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 95/5) gereinigt.

Ausbeute: 880 mg weißer Feststoff (87%), Smp. = 142-144°C.

³¹P-NMR (CD₂Cl₂): d +126.9 ppm (52%) und +126.6 ppm (48%), Gemisch aus (*R,R*)/(*S,S*)*-* und (*R*,*S*)-Verbindung.

¹H-NMR (CD₂Cl₂): *Diastereomer A,* d 7.35 (2H, s, 2xHₐᵣ), 7.09 (2H, s, 2xHₐᵣ), 6.96 (2H, d, *J* 2.2 Hz, 2xHₐᵣ), 5.90 (2H, d, *J* 2.1 Hz, 2xHₐᵣ), 2.30 (6H, s, 2xCH₃), 2.23 (6H, s, 2xCH₃), 1.80 (6H, s, 2xCH₃), 1.75 (6H, s, 2xCH₃), 1.53 (18H, s, 2xC(CH₃)₃), 1.17 (18H, s, 2xC(CH₃)₃), 1.09 (18H, s, 2xC(CH₃)₃). *Diastereomer B,* d 7.26 (2H, s, 2xHₐᵣ), 7.12 (2H, s, 2xHₐᵣ), 7.08 (2H, d, *J* 2.2 Hz, 2xHₐᵣ), 6.45 (2H, d, *J* 2.1 Hz, 2xHₐᵣ), 2.29 (6H, s, 2xCH₃), 2.25 (6H, s, 2xCH₃), 1.83 (6H, s, 2xCH₃), 1.82 (6H, s, 2xCH₃), 1.51 (6H, s, 2xCH₃), 1.22 (18H, s, 2xC(CH₃)₃), 1.14 (18H, s, 2xC(CH₃)₃).

¹³C-NMR (CD₂Cl₂): Diastereomerengemisch, Cₐᵣ-Signale von 146-129 ppm nicht zuordbar; 128.8 (2xCₐᵣH), 128.7 (2xCₐᵣH), 128.1 (4xCₐᵣH), 119.7 (2xCₐᵣH), 118.6 (2xCₐᵣH), 118.0 (2xCₐᵣH), 117.7 (2xCₐᵣH), 35.3 (2x^{t}C), 35.2 (^{t}C),34.9 (^{t}C), 34.8 (^{t}C), 34.7 (^{t}C), 34.6 (^{t}C), 34.6 (^{t}C), 33.8 (2xCH₃), 33.6 (2xCH₃), 31.9 (6xCH₃), 31.8 (6xCH₃), 31.5 (m, 6xCH₃), 31.3 (6xCH₃), 31.2 (6xCH₃), 31.1 (m, 6xCH₃), 20.7 (2xCH₃), 20.6 (2xCH₃), 20.5 (2xCH₃), 20.5 (2xCH₃), 16.9 (2xCH₃), 16.8 (2xCH₃), 16.2 (4xCH₃).

HRMS (ESI-TOF) [M+H]⁺: m/z berechnet: für C₇₁H₉₂O₇P₂ 1119.6396, gefunden: 1119.6414.

HRMS (ESI-TOF) [M+Na]⁺: m/z berechnet: für C₇₁H₉₂O₇P₂ 1141.6211, gefunden: 1141.6232.

### 6,6'-((2,7-Di-tert-butyl-9,9-dimethyl-9H-xanthene-4,5-diyl)bis(oxy))didibenzo[d,f][1,3,2]dioxa-phosphepin (Verbindung 16)

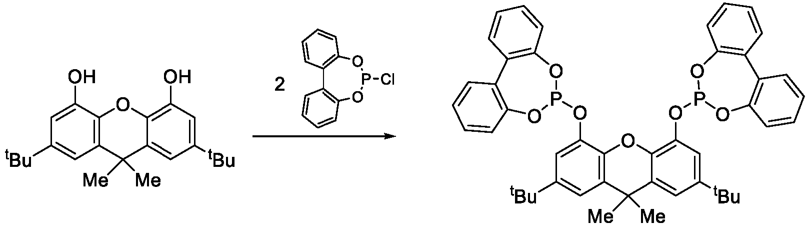

Das 2,7-Di-*tert*-butyl-9,9-dimethyl-9*H*-xanthene-4,5-diol (328 mg, 0.93 mmol) und Triethylamin (281 mg, 2.77 mmol) in Toluol (4 ml) wird bei 0 °C mit 6-Chlorodibenzo[*d*,*f*][1,3,2]dioxaphosphepin (510 mg, 2.04 mmol) in Toluol (5 ml) tropfenweise versetzt. Nach dem Rühren über Nacht wird vom ausgefallenen Niederschlag abfiltriert und das Lösemittel unter Vakuum entfernt. Der verbleibende Rückstand wird aus Acetonitril umkristallisiert und nach Filtration und Trocknen das Bisphosphit mit einer Reinheit von 95% (³¹P-NMR) erhalten.

Ausbeute: 480 mg weißer Feststoff (66%).

³¹P-NMR (CD₂Cl₂): d +139.8 ppm.

¹H-NMR (CD₂Cl₂): ): d 7.51-7.46 (4H, m, 4xHₐᵣ), 7.35-7.21 (14H, m, 14xHₐᵣ), 7.13 (2H, m, 2xHₐᵣ), 1.72 (6H, s, 2xCH₃), 1.30 (18H, s, 2xC(CH₃)₃).

¹³C-NMR (CD₂Cl₂): d 149.7 (m, 4xCₐᵣ), 146.5 (4xCₐᵣ),139.8 (2xCₐᵣ), 139.1 (2xCₐᵣ), 131.5 (2xCₐᵣ), 131.2 (2xCₐᵣ), 130.2 (4xCₐᵣH), 129.6 (4xCₐᵣH), 125.7 (4xCₐᵣH), 122.7 (4xCₐᵣH), 119.0 (2xCₐᵣH), 118.7 (m, 2xCₐᵣH), 35.4 (^{t}C), 34.9 (2x^{t}C), 32.9 (2xCH₃), 31.5 (6xCH₃).

HRMS (ESI-TOF) [M+2O+H]⁺: m/z berechnet: für C₄₇H₄₄O₇P₂ 815.2534, gefunden: 815.2551

HRMS (ESI-TOF) [M+2O+Na]⁺: m/z berechnet: für C₄₇H₄₄O₇P₂ 837.2352, gefunden: 837.2371.

### 6,6'-((9,9-Dimethyl-9H-xanthen-4,5-diyl)bis(oxy))bis(4,8-di-tert-butyl-2,10-dimethoxy-dibenzo[d,f][1,3,2]dioxaphosphepin) (Verbindung 17)

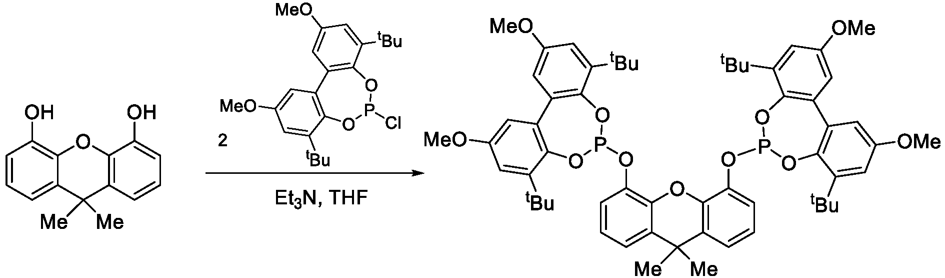

Zu einer Lösung von 9,9-Dimethyl-9*H*-xanthen-4,5-diol (127 mg, 0.52 mmol) und Triethylamin (161 mg, 1.59 mmol) in THF (2 ml) wird unter Rühren und bei 0 °C das 4,8-Di-tert-butyl-6-chlor-2,10-dimethoxydibenzo[*d*,*f*][1,3,2]dioxaphosphepin (445 mg, 1.05 mmol) in THF (5 ml) langsam zugetropft und man lässt anschließend bei Raumtemperatur über Nacht weiterrühren. Zur Aufarbeitung wird vom ausgefallenem Hydrochlorid abfiltriert und das Lösemittel unter Vakuum entfernt. Zur weiteren Reinigung wurde der feste Rückstand mit Toluol (6 ml) aufgenommen und die unlöslichen Bestandteile abgetrennt. Nach dem Entfernen des Lösemittels und Trocknen wird das Produkt mit sehr guter Reinheit erhalten.

Ausbeute: 365 mg weißer Feststoff (68%).

³¹P-NMR (CD₂Cl₂): d +136.6 ppm.

¹H-NMR (CD₂Cl₂): d 7.03 (1H, dd, *J* 8.0, 1.5 Hz, 2xHₐᵣ), 7.01 (4H, d, *J* 3.1 Hz, 4xHₐᵣ), 6.76 (2H, t, *J* 8.0 Hz, 2xHₐᵣ), 6.70 (4H, d, *J* 3.1 Hz, 4xHₐᵣ), 6.05 (2H, br d, *J* 3.0 Hz, 2xHₐᵣ), 3.81 (12H, s, 4xOCH₃), 1.52 (6H, s, 2xCH₃), 1.38 (36H, s, 4xC(CH₃)₃).

¹³C-NMR (CD₂Cl₂): d 156.3 (4xCₐᵣ-OMe), 143.1 (4xCₐᵣ), 142.0 (2xCₐᵣ), 140.6 (2xCₐᵣ),134.2 (4xCₐᵣ), 130.7 (2xCₐᵣ), 129.4 (2xCₐᵣ), 128.6 (2xCₐᵣ), 122.8 (2xCₐᵣH), 121.9 (2xCₐᵣH), 120.7 (2xCₐᵣH), 114.6 (4xCₐᵣH), 113.3 (4xCₐᵣH), 56.0 (4xOCH₃), 35.6 (4x^{t}C), 34.3 (^{t}C), 33.8 (2xCH₃), 31.0 (12xCH₃).

HRMS (ESI-TOF) [M+H]⁺: m/z berechnet: für C₅₉H₆₈O₁₁P₂ 1015.4315, gefunden1015.4313.

| | | | | |
|---|---|---|---|---|
| Elementaranalyse: | berechnet: | C 69.81% | H 6.75% | P 6.10% |
| | gefunden: | C 69.66% | H 6.90% | P 6.02% |

### (4S,4'S,5R,5'R)-2,2'-((9,9-Dimethyl-9H-xanthen-4,5-diyl)bis(oxy))bis(4,5-diphenyl-1,3,2-dioxa-phospholan) (Verbindung 18)

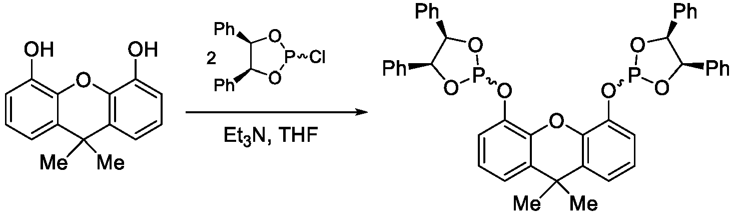

Zu einer Lösung von 9,9-Dimethyl-9*H*-xanthen-4,5-diol (228 mg, 0.94 mmol) und Triethylamin (285 mg, 2.82 mmol) in THF (3 ml) wird unter Rühren und bei 0 °C das (4*R*,5*S*)-2-Chlor-4,5-diphenyl-1,3,2-dioxaphospholan (524 mg, 1.88 mmol) in THF (6 ml) langsam zugetropft und man lässt anschließend bei Raumtemperatur über Nacht weiterrühren. Zur Aufarbeitung wird vom ausgefallenem Hydrochlorid abfiltriert und das Lösemittel unter Vakuum entfernt. Das Produkt liegt bereits mit guter Reinheit vor und fällt als ein Gemisch aus den verschieden cis-/trans-Konfigurationen im 1,3,2-Dioxophospholan an.

Ausbeute: 365 mg weißer Feststoff (68%).

³¹P-NMR (CD₂Cl₂): d +141.1 ppm und +132.2 ppm (63%, 2xd, *J_{P,P}* 6.1 Hz, cis,trans-Produkt), +140.7 ppm (9%, *cis*,*cis*-Produkt), +132.9 ppm (26%, *trans*,*trans*-Produkt).

¹H-NMR (CD₂Cl₂): d 7.34-7.00 (22H, m 22xHₐᵣ), 6.96-6.90 (2H, m, 2xHₐᵣ), 6.84-6.70 (2H, m, 2xHₐᵣ), 6.01 (4H, br s, 4xCH-O_{trans,trans}), 5.78 (2H, d, *J 1.7* Hz, 2xCHO_{cis,trans}), 5.74 (2H, d, *J* 8.7 Hz, 2xCHO_{cis,trans}), 5.69 (4H, m, 4xCHO_{cic,cis}), 1.74 (6H, s, 2xCH_{3,cic,cis}), 1.72 (6H, s, 2xCH_{3,cis,trans}), 1.71 (6H, s, 2xCH₃, _{trans,trans}).

HRMS (ESI-TOF) [M+H]⁺: m/z berechnet: für C₄₃H₃₆O₇P₂ 727.2014, gefunden: 727.2017.

| | | | | |
|---|---|---|---|---|
| Elementaranalyse: | berechnet: | C 71.07% | H 4.99% | P 8.53% |
| | gefunden: | C 70.78% | H 5.04% | P 8.75% |

### 6,6'-((9,9-Dimethyl-9H-xanthen-4,5-diyl)bis(oxy))bis(2,4,8,10-tetra-tert-butyldibenzo[d,f][1,3,2]dioxa-phosphepin) (Verbindung 19)

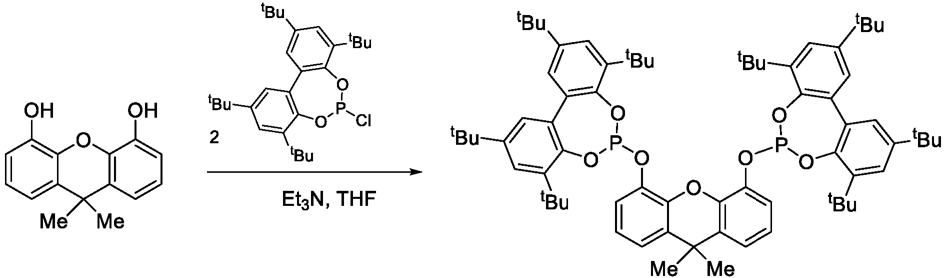

Zu einer Lösung von 9,9-Dimethyl-9*H*-xanthen-4,5-diol (125 mg, 0.52 mmol) und Triethylamin (156 mg, 1.55 mmol) in THF (2 ml) wird unter Rühren und bei 0 °C das 2,4,8,10-Tetra-*tert*-butyl-6-chlorodibenzo[*d*,*f*][1,3,2]dioxaphosphepin (490 mg, 1.04 mmol) in THF (5 ml) langsam zugetropft und man lässt anschließend bei Raumtemperatur über Nacht weiterrühren. Zur Aufarbeitung wird vom ausgefallenem Hydrochlorid abfiltriert und das Lösemittel unter Vakuum entfernt. Zur Verbesserung der Reinheit wird das Produkt mit heißem Heptan suspendiert, nach dem Abkühlen abgetrennt und getrocknet.

Ausbeute: 310 mg weißer Feststoff (54%).

³¹P-NMR (CD₂Cl₂): d +137.8 ppm.

¹H-NMR (CD₂Cl₂): d 7.48 (4H, m, 4xHₐᵣ), 7.13 (4H, d, *J* 2.5 Hz, 4xHₐᵣ), 6.96 (2H, dd, *J* 8.0, 1.4 Hz, 2xHₐᵣ), 6.61 (2H, t, *J* 8.0 Hz, 2xHₐᵣ), 5.69 (2H, d, *J* 7.8 Hz, 2xHₐᵣ), 1.47 (6H, s, 2xCH₃), 1.40 (36H, br s, 4xC(CH₃)₃), 1.37 (36H, s, 4xC(CH₃)₃).

¹³C-NMR (CD₂Cl₂): d 147.4 (4xCₐᵣ), 146.3 (2xCₐᵣ), 140.8 (4xCₐᵣ), 140.8 (2xCₐᵣ), 140.7 (4xCₐᵣ), 133.3 (4xCₐᵣ), 130.3 (2xCₐᵣ), 127.0 (4xCₐᵣH), 124.7 (4xCₐᵣH), 122.5 (2xCₐᵣH), 121.9 (2xCₐᵣH), 120.7 (2xCₐᵣH), 36.9 (4x^{t}C), 35.0 (4x^{t}C), 34.2 (^{t}C), 33.9 (2xCH₃), 31.7 (12xCH₃), 31.1 (12xCH₃).

HRMS (ESI-TOF) [M+H]⁺: m/z berechnet: für C₇₁H₉₂O₇P₂ 1119.6396, gefunden: 1119.6398.

| | | | | |
|---|---|---|---|---|
| Elementaranalyse: | berechnet: | C 76.18% | H 8.28% | P 5.54% |
| | gefunden: | C 76.10% | H 8.31% | P 5.52% |

### 5-((rac-4,8-Di-tert-butyl-1,2,10,11-tetramethyldibenzo[d,f][1,3,2]dioxaphosphepin-6-yl)oxy)-9,9-dimethyl-9H-xanthen-4-ol

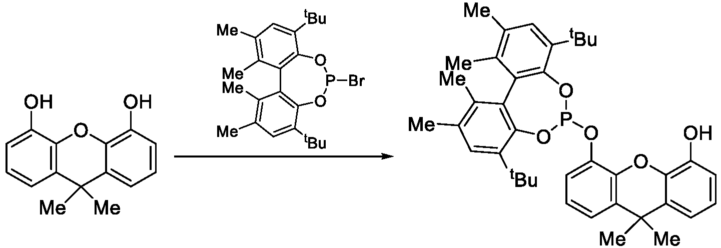

Das *rac*-6-Bromo-4,8-di-*tert*-butyl-1,2,10,11-tetramethyldibenzo[*d*,*f*][1,3,2]dioxaphosphepin (760 mg, 1.64 mmol) in Toluol (5 ml) wird langsam bei 0°C zu einer Lösung von 1.2 Äquivalenten 9,9-Dimethyl-9H-xanthene-4,5-diol (477 mg, 1.97 mmol) und Triethylamin (498 mg, 4.92 mmol) in Toluol (10 ml) getropft. Nach 30 Minuten lässt man über Nacht bei Raumtemperatur weiterrühren und filtriert anschließend vom ausgefallenen Niederschlag ab. Nach dem Einengen unter Vakuum wurde der Rückstand mit Acetonitril zur Kristallisation gebracht und abfiltriert. Das erhaltene Rohprodukt enthält trotz leichtem Überschuss an Diol ca. 15 mol-% der disubstituierten Verbindung. Zur weiteren Reinigung wurde die Zielverbindung durch mehrfache Säulenchromatographie (Eluent: Cyclohexan/AcOEt 99/1 bis 95/5) mit einer Reinheit >95% (³¹P-NMR) isoliert.

Ausbeute: 370 mg weißer Feststoff (36%), Smp. = 177-178°C.

³¹P-NMR (CD₂Cl₂): d +133.3 ppm.

¹H-NMR (CD₂Cl₂): d 7.29 (1H, s, Hₐᵣ), 7.18 (1H, m, Hₐᵣ), 7.17 (1H, s, Hₐᵣ), 6.98 (1H, d, *J* 7.9 Hz, Hₐᵣ), 6.95 (1H, d, *J* 7.8 Hz, Hₐᵣ), 6.92 (1H, d, *J* 8.0, 2.0 Hz, Hₐᵣ), 6.77 (1H, dd, *J* 7.4, 2.1 Hz, Hₐᵣ), 6.74 (1H, m, Hₐᵣ), 5.71 (1H, d, *J* 1.8 Hz, OH), 2.33 (3H, s, CH₃), 2.27 (3H, s, CH₃), 1.90 (3H, s, CH₃), 1.84 (3H, s, CH₃), 1.61 (3H, s, CH₃), 1.58 (3H, s, CH₃), 1.49 (9H, s, C(CH₃)₃), 1.25 (9H, s, C(CH₃)₃).

¹³C-NMR (CD₂Cl₂): d 144.8 (d, *J* 5.4 Hz, Cₐᵣ), 144.7 (Cₐᵣ), 144.6 (d, *J* 2.6 Hz, Cₐᵣ), 141.8 (d, *J* 2.0 Hz, Cₐᵣ), 139.8 (Cₐᵣ), 138.6 (d, *J* 2.7 Hz, Cₐᵣ), 138.0 (Cₐᵣ), 138.0 (Cₐᵣ), 135.7 (Cₐᵣ), 135.1 (Cₐᵣ), 133.6 (Cₐᵣ), 132.9 (Cₐᵣ), 132.5 (Cₐᵣ), 132.3 (d, *J* 4.8 Hz, Cₐᵣ), 130.9 (d, *J* 3.5 Hz, Cₐᵣ), 130.9 (Cₐᵣ), 128.7 (CₐᵣH), 128.4 (CₐᵣH), 123.8 (CₐᵣH), 123.4 (CₐᵣH), 121.9 (CₐᵣH), 120.0 (d. *J* 6.3 Hz, CₐᵣH), 117.0 (CₐᵣH), 113.4 (CₐᵣH), 35.2 (^{t}C), 34.9 (^{t}C), 34.8 (^{t}C), 32.3 (CH₃), 32.0 (CH₃), 31.7 (3xCH₃), 31.3 (d, *J* 5.1 Hz, 3xCH₃), 20.5 (2xCH₃), 16.8 (CH₃), 16.6 (CH₃).

HRMS (ESI-TOF) [M+H]⁺: m/z berechnet: für C₃₉H₄₅O₅P 625.3083, gefunden: 625.3081.

HRMS (ESI-TOF) [M+Na]⁺: m/z berechnet: für C₃₉H₄₅O₅P 647.2897, gefunden: 647.2902.

### rac-4,8-Di-tert-butyl-6-((9,9-dimethyl-5-((2,4,8,10-tetra-tert-butyldibenzo[d,f][1,3,2]dioxaphos-phepin-6-yl)oxy)-9H-xanthen-4-yl)oxy)-1,2,10,11-tetramethyldibenzo[d,f][1,3,2]dioxaphosphepin (Verbindung 20)

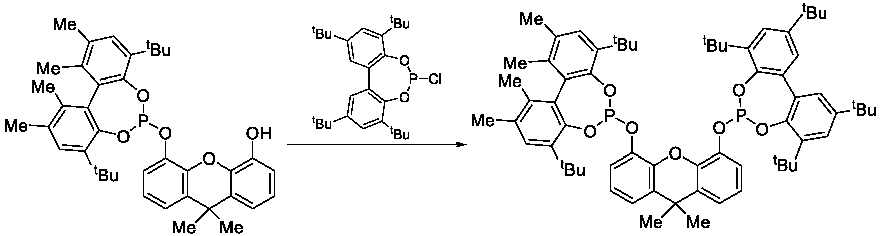

Zu einer Lösung des 5-((*rac*-4,8-Di-*tert*-butyl-1,2,10,11-tetramethyldibenzo[*d*,*f*] [1,3,2]dioxaphosphepin-6-yl)oxy)-9,9-dimethyl-9*H*-xanthen-4-ols (240 mg, 0.38 mmol) in Toluol (4 ml) gibt man bei 0 °C zunächst Triethylamin (58 mg, 0.58 mmol) und anschließend eine Lösung des 2,4,8,10-Tetra-*tert*-butyl-6-chlorodibenzo[*d*,*f*][1,3,2]dioxaphosphepins (201 mg, 0.42 mmol) in Toluol (2 ml) hinzu. Nach 30 Minuten lässt man auf Raumtemperatur erwärmen und rührt über Nacht weiter. Nach der Filtration und einengen des Filtrats wird der verbleibende Rückstand durch Säulenchromatographie (Eluent: Cyclohexan/AcOEt 99/1 bis 95/5) in hoher Reinheit erhalten.

Ausbeute: 210 mg weißer Feststoff (59%), Smp. = 152-154°C.

³¹P-nmr (CD₂Cl₂): d +137.8 ppm (d, *J* 56.5 Hz) und +128.5 ppm (d, *J* 56.5 Hz) ppm.

¹H-NMR (CD₂Cl₂): d 7.56 (1H, d, *J* 2.4 Hz, Hₐᵣ), 7.33 (1H, d, *J* 2.5 Hz, Hₐᵣ), 7.32 (2H, s, 2xHₐᵣ), 7.13 (1H, d, *J* 2.4 Hz, Hₐᵣ), 7.09 (1H, d, *J* 2.4 Hz, Hₐᵣ), 7.09 (H, s, Hₐᵣ), 6.97 (2H, m, 2xHₐᵣ), 6.66 (1H, dd, *J* 8.0, 8.0 Hz, Hₐᵣ), 6.60 (1H, dd, *J* 8.0, 8.0 Hz, Hₐᵣ), 5.76 (1H, d, *J* 7.8 Hz, Hₐᵣ), 5.67 (1H, d, *J* 7.8 Hz, Hₐᵣ), 2.33 (3H, s, CH₃), 2.23 (3H, s, CH₃), 1.78 (3H, s, CH₃), 1.78 (3H, s, CH₃), 1.52 (9H, s, C(CH₃)₃), 1.49 (9H, s, C(CH₃)₃), 1.49 (3H, s, CH₃), 1.47 (3H, s, CH₃), 1.41 (9H, s, C(CH₃)₃), 1.29 (9H, s, C(CH₃)₃), 1.23 (9H, s, C(CH₃)₃), 1.18 (9H, s, C(CH₃)₃).

¹³C-NMR (CD₂Cl₂): d 147.5 (Cₐᵣ), 147.1 (Cₐᵣ), 146.2-130.3 (22xCₐᵣ), 128.4 (CₐᵣH), 128.0 (CₐᵣH), 126.9 (2xCₐᵣH), 124.8 (CₐᵣH), 124.4 (CₐᵣH), 122.5 (2xCₐᵣH), 121.9 (CₐᵣH), 121.6 (CₐᵣH), 120.8 (CₐᵣH), 120.7 (CₐᵣH), 35.8 (^{t}C), 35.4 (^{t}C), 35.2 (^{t}C), 35.1 (^{t}C), 34.9 (^{t}C), 34.7 (^{t}C), 34.3 (^{t}C), 34.1 (CH₃), 33.4 (CH₃), 31.8 (3xCH₃), 31.7 (3xCH₃), 31.6 (3xCH₃), 31.4 (3xCH₃), 31.0 (d, *J* 5.2 Hz, 3xCH₃), 30.8 (d, *J* 3.9 Hz, 3xCH₃), 20.5 (CH₃), 20.5 (CH₃), 16.7 (CH₃), 16.6 (CH₃).

HRMS (ESI-TOF) [M+H]⁺: m/z berechnet: für C₆₇H₈₄O₇P 1063.5770, gefunden: 1063.5768.

HRMS (ESI-TOF) [M+Na]⁺: m/z berechnet: für C₆₇H₈₄O₇P 1085.5585, gefunden: 1085.5588.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Cis/trans-2-Penten (Aldrich) wurde über Natrium am Rückfluss erhitzt und unter Argon destilliert. Im Autoklav wurden unter Argonatmosphäre Lösungen der Katalysatorvorstufe und des Liganden jeweils in Toluol gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischung wurden entnommen, mit 10 ml Pentan verdünnt und gaschromatographisch analysiert: HP 5890 Series II plus, PONA, 50 m x 0,2 mm x 0,5 µm.

Die Ergebnisse der Versuchsreihe sind in der nachfolgenden Tabelle aufgelistet:

| Ligand | Ausbeute [%] |
|---|---|
| **(1)** | 99 |
| **(2)** | 99 |
| **(3)** | 99 |
| **(4)** | 99 |
| **(5)** | 99 |
| **(6)** | 99 |
| **(7)** | 99 |
| **(8)** | 99 |
| **(9)** | 98 |
| **(10)** | 99 |
| **(11)** | 52 |
| **(12)** | 97 |
| **(13)** | 94 |
| **(14)** | 42 |
| **(15)** | 99 |
| **(16)** | 99 |
| **(17)** | 99 |
| **(18)** | 97 |
| **(19)** | 99 |
| **(20)** | 98 |

Die durchgeführten Versuche belegen, dass die gestellte Aufgabe durch die erfindungsgemäßen Verbindungen gelöst wird.

## Patentansprüche

1. Verbindung gemäß einer der Formeln **(1)** bis **(20):**
